# EUROPEAN PATENT APPLICATION

(11) **EP 2 168 595 A1**
(43) Date of publication of application: **31.03.2010**
(21) Application number: 08757901.7
(22) Date of filing: 16.06.2008
(51) Int. Cl.: A61K 39/095, A61P 31/04

(54) **PHARMACEUTICAL COMPOSITIONS CONTAINING THE PROTEIN NMB1796**

(30) Priority: 27.06.2007 CU 20070151
(71) Applicant: Centro De Ingenieria Genetica Y Biotecnologia, Ciudad De La Habana 10600 (CU)
(72) Inventor: PAJÓN FEYT, Rolando, Ciudad De La Habana 10700 (CU); NIEBLA PEREZ, Olivia, Ciudad De La Habana 11600 (CU); SARDIÑAS GARCIA, Gretel, Ciudad De La Habana 10500 (CU); GONZALEZ BLANCO, Sonia, Ciudad De La Habana 11600 (CU); GARCIA DIAZ, Darién, Ciudad De La Habana 11300 (CU); CABALLERO MENENDEZ, Evelin, Ciudad De La Habana 11600 (CU); COBAS ACOSTA, Karem, Ciudad De La Habana 19100 (CU)
(74) Representative: Hatzmann, Martin
(86) International application number: PCT/CU2008/000004
(87) International publication number: WO 2009/000217

(57) **Abstract**

The present invention is related to field of medicine, particularly to the development of pharmaceutical composition containing the NMB1796 protein. The composition described in this invention are able to confer protection against different diseases caused or not by pathogenic agents. The NMB1796 protein was identified as a *Neisseria meningitidis* outer membrane vesicle (OMV) component, and it was obtained through recombinant DNA technology being its immunogenicity and protective activity evaluated in animal models. Due to the high level of conservation that the NMB1796 coding gene has shown, pharmaceutical compositions containing this protein have a high value as inducers of a cross-reactive immune response. The composition presented in this invention is applicable to the field of human medicine.

## Description

### Technical Field

The present invention is related to field of medicine, particularly to the development of new vaccine composition of preventive or therapeutic application, that allow an increase in the quality of immune response against vaccine antigens of diseases from different sources.

### Previous Art

*Neisseria meningitidis,* a Gram-negative diplococcus whose only know host is man, is the causal agent of meningococcal meningitis. Usually this bacterium is found in asymptomatic carriers among the normal population, being this niche the most common source for its microbiological isolation.

On world basis, small children less than two years of age are the more susceptible population for contracting meningococcal meningitis, however, young adults and normal adult population may also be affected.

Untreated meningococcal disease has a fatal course for most affected individuals, and vaccination could prevent this situation, by halting the events as early as at the bacterial colonization phase.

Several strategies have been developed with the aim of obtaining a vaccine able to fulfill the needed requirements in order to induce protection against this disease in general population. For this purpose capsular antigens have been taken into account, since their immunological specificity has allowed the classification into serogroups of this microorganism. Five of these serogroups have been defined as responsible of most of the clinical cases of meningococcal disease all around the world. Serogroup A is the principal cause of epidemics in sub-Saharan Africa. Serogroups B and C are associated, in most cases, to the occurrences in developed nations. Serogroups Y and W135 are common in most of the recurrent cases of the disease, and they are prevalent in some areas of USA, with a marked increase in last few years. From this data it is obvious the reason of the use, study, and evaluation of capsular polysaccharides as vaccine candidates. A tetravalent vaccine, based on capsular polysaccharides, conferring protection against serogroups A, C, Y, and W-135 has been licensed in Unite States. Elicited antibodies after vaccination are serogroup-specific (Rosenstein N. et al. 2001. Menningococcal disease. N. Engl. J. Med, 344, 1378-1388).

Serogroup B, which is different from the rest, continues to be a significant cause of endemic and epidemic meningococcal disease, and thi is mainly due to the complete lack of efficient vaccines against it. It has been noted that B capsular polysaccharide is poorly immunogenic, plus the existence of the theoretical risk for a vaccine based on this compound to induce immuno-tolerance and autoimmunity because of its structural similarity to oligosaccharide chains that are present in human neural fetal structures. (Finne J. et al. 1987. An IgG monoclonal antibody to group B meningococci cross-reacts with developmentally regulated polysialic acid units of glycoproteins in neural and extraneural tisues. J. Immunol, 138: 4402-4407). Therefore, the development of vaccines against serogroups B is concentrated in the use of sub-capsular antigens.

### Outer membrane proteins and vesicle vaccines

Initial attempts, in the 70s, to produce vaccines based on outer membrane proteins were based on the LPS depletion of outer membrane protein preparations by detergent (Frasch CE and Robbins JD. 1978. Protection against group B meningococcal disease. III. Immunogenicity of serotype 2 vaccines and specificity of protection in a guinea pig model. J Exp Med 147(3):629-44). The outer membrane proteins, OMPs, were then precipitated to produce aggregates suspended in sodium chloride. Despite promising results in animal studies, these vaccines failed to induce bactericidal antibody in either adults or children (Zollinger WD, et al. 1978. Safety and immunogenicity of a Neisseria meningitidis type 2 protein vaccine in animals and humans. J. Infect. Dis. 137(6):728-39), The poor performance of these vaccines was largely attributed to the loss of tertiary structure that accompanied precipitation. The next logical step was, therefore, to produce a vaccine with proteins displayed in their native conformation in the form of vesicles of outer membrane (Zollinger WD, et al. 1979. complex of meningococcal group B polysaccharide and type 2 outer membrane protein immunogenic in man. J. Clin. Invest. 63(5):836-48, Wang LY and Frasch CE. 1984. Development of a Neisseria meningitidis group B serotype 2b protein vaccine and evaluation in a mouse model. Infect Immun. 46(2):408-14136).

These outer membrane vesicle vaccines were significantly more immunogenic than the OMP aggregates and immunogenicity was shown to be further enhanced by adsorption to the adjuvant aluminium hydroxide (Wang LY and Frasch CE. 1984. Neisseria meningitidis group B serotype 2b protein vaccine and evaluation in a mouse model. Infect Immun. 46(2):408-14136).

A number of efficacy trials have been carried out using soluble outer membrane vesicle vaccines of different formulations. The two vaccines most extensively studied were developed in the 1980s in response to outbreaks of disease in Cuba (Sierra GV et al. 1991. Vaccine against group B Neisseria meningitidis: protection trial and mass vaccination results in Cuba. NIPH Ann Dis. 14(2):195-210) and Norway (Bjune G, et al. 1991. Effect of outer membrane vesicle vaccine against group B meningococcal disease in Norway. Lancet. 338(8775):1093-6), respectively. The OMV vaccine produced by the Finlay Institute in Cuba (commercially marketed as VA-MENGOC-BC ) is produced from strain B:4:P1.19,15 with serogroup C polysaccharide and a preparation of high molecular weight OMPs and is adsorbed to aluminium hydroxide (Sierra GV et al. 1991. Vaccine against group B Neisseria meningitidis: protection trial and mass vaccination results in Cuba. NIPH Ann Dis. 14(2):195-210). This vaccine contributed to the rapid decline of the epidemic in Cuba (Rodriguez AP, et al. The epidemiological impact of antimeningococcal B vaccination in Cuba.1999. Mem Inst Oswaldo Cruz. 94(4):433-40).

The vaccine produced by the Norwegian National Institute for Public Health (NIPH) was similarly intended initially for use during a period of hyperendemic disease caused by another organism from the ET-5 clone (B:15:P1.7,16). It was also a monovalent vaccine produced from purified outer membrane vesicles adsorbed onto aluminium hydroxide (BjuneG, et al. 1991. Effect of outer membrane vesicle vaccine against group B meningococcal disease in Norway. Lancet. 338(8775):1093-6). Outer membrane vesicle vaccines appear to effectively present outer membrane proteins in a sufficiently natural conformation to allow the generation of functional bactericidal antibodies, at least in teenagers and adults. The antibody responses generated have also been shown to increase opsonophagocytosis of meningococci. The precise formulation of the vaccines (i.e. OMP content, LPS content and the presence or absence of adjuvant) have a significant impact on immunogenicity (Lehmann AK, et al. 1991. Immunization against serogroup B meningococci. Opsonin response in vaccinees as measured by chemiluminescence.APMIS. 99(8):769-72, Gomez JA, et al. 1998. Effect of adjuvants in the isotypes and bactericidal activity of antibodies against the transferrin-binding proteins of Neisseria meningitidis. Vaccine. 16(17):1633-9, Steeghs L, et al. 1999. Immunogenicity of Outer Membrane Proteins in a Lipopolysaccharide-Deficient Mutant of Neisseria meningitidis: Influence of Adjuvants on the Immune Response. Infect Immun. 67(10):4988-93).

The antigenic profile of disease isolates also changes rapidly and a vaccine with coverage of only a limited number of selected strains is likely to become ineffective within a few years unless the vaccine composition is changed to mirror local epidemiology.

At present OMV vaccines have been used more widely than any other serogroup B vaccine and are potentially useful in the context of outbreaks of disease caused by a single PorA type.

The immunogens that generate cross-reactivity between strains have yet to be fully defined. Studies of post-vaccination sera from both Finlay Institute and NIPH vaccine trials suggested that antibodies against both PorA (P1, the class 1 serosubtype protein) and OpcA (another major OMP, formerly known as Opc) (Wedege E, et al. 1998. Immune Responses against Major Outer Membrane Antigens of Neisseria meningitidis in Vaccinees and Controls Who Contracted Meningococcal Disease during the Norwegian Serogroup B Protection Trial. Infect Immun. 66(7): 3223-31), were both important in the mediation of serum bactericidal activity (wilh PorA mosl immunogenic) both these antigens show marked strain to strain variability.

The prominence of PorA protein and the significant level of variability in this protein, which appears to undergo continuous variation both between and during oulbreaks (Jelfs J, et al. 2000. Sequence Variation in the porA Gene of a Clone of Neisseria meningitidis during Epidemic Spread. Clin Diagn Lab Immunol. 7(3):390-5) in epitopes to which most of the bactericidal activity in post-vaccination (and post-disease) is directed enhanced concerns that protection offered by single strain (monovalent) OMV-based vaccines might be serosubtype restricted (i.e. dependent on The PorA type).

In an attempt to overcome this potential problem, an OMV vaccine was developed in The Netherlands at RIVM that contained PorA proteins from six different prevalent pathogenic isolates (Van Der Ley P and Poolman JT. 1992. Construction of a multivalent meningococcal vaccine strain based on the class 1 outer membrane protein. Infect Immun. 60(8):3156-61, Claassen I, et al. 1996. Production, characterization and control of a Neisseria meningitidis hexavalent class 1 outer membrane protein containing vesicle vaccine. Vaccine. 14(10):1001-8). In this case the vaccine vesicles were extracted from two variants of the well-characterized H44/76 strain which had been genetically engineered lo express three separate PorA proteins.

### The search for a universal antigen

It is clear that outer membrane proteins (OMP) can induce a functional immune response against serogroup B disease but that none of the vaccines so far developed are universally protective due to the great heterogeneity of the surface exposed regions of the outer membrane proteins. The modest cross-reactive immunity induced by the outer membrane vesicles (OMV) vaccines has fuelled the search for an outer membrane antigen (or group of antigens), which induces functional antibodies and which is present on all meningococcal strains. Such antigens, if they were present on all strains irrespective of serogroup, might form the basis of a truly universal meningococcal vaccine, which would eliminate the potential problem of capsular switching on pathogenic strains following polysaccharide vaccination.

Once it became apparent that the variability of the immunodominant PorA protein would limit its use as a universal vaccine, a number of the other major outer membrane proteins were considered for their vaccine potential and several of these are under further development. Those which have been considered include class 5 proteins (OpcA), NspA and iron regulated proteins (TbpA and B, FbpA, FetA). TbpB forms part of the transferrin binding complex with TbpA. Recent work suggests that TbpA has both a greater functional role in iron binding (Pintor M, et al. 1998. Analysis of TbpA and TbpB functionality in defective mutants of Neisseria meningitidis.J Med Microbiol 47(9): 757-60) and is a more effective immunogen than TbpB.

A highly conserved minor outer membrane protein has been discovered via a novel technique using combinations of outer membrane protein preparations from different meningococcal strains to immunize mice (Martin D, et al. 1997. Highly Conserved Neisseria meningitidis Surface Protein Confers Protection against Experimental Infection. J Exp Med 185 (7): 1173-83). The B cells from the mice were used to produce hybridomas which were then screened for cross-reactivity against multiple strains of meningococci. One highly cross-reactive monoclonal antibody was found to bind to a 22 kDa outer membrane protein that was designated NspA. Immunization with recombinant NspA protein was shown to induce a cross-reactive bactericidal response in mice against strains from serogroups A-C. Vaccination also protects mice against lethal meningococcal infection (Martin D, et al. 1997. Highly Conserved Neisseria meningitidis Surface Protein Confers Protection against Experimental Infection. J Exp Med 185 (7): 1173-83). Comparison of NspA sequences among genetically divergent meningococcal strains demonstrates that the protein is highly conserved (97% homology) (Cadieux N, et al. 1999. Bactericidal and Cross-Protective Activities of a Monoclonal Antibody Directed against Neisseria meningitidis NspA Outer Membrane Protein. Infect Immun 67 (9): 4955-9). The presence of NspA was detected by ELISA on 99.2% of tested strains from serogroups A-C using anti-NspA monoclonal antibodies (Martin D, et al. 1997. Highly Conserved Neisseria meningitidis Surface Protein Confers Protection against Experimental Infection. J Exp Med 185 (7): 1173-83). These monoclonal antibodies have been shown to be bactericidal against numerous strains of meningococci and are able to reduce meningococcal bacteraemia in a mouse model (Cadieux N, et al. 1999. Bactericidal and Cross-Protective Activities of a Monoclonal Antibody Directed against Neisseria meningitidis NspA Outer Membrane Protein. Infect Immun 67 (9): 4955-9). Although this data appears to suggest that NspA is a promising vaccine candidate that is able to protect across serogroup boundaries, polyclonal anti-recombinant NspA serum from mice does not bind to the surface of around 35% of pathogenic serogroup B meningococcal strains despite the presence of the *nspA* gene in these organisms (Moe GR et al. 1999. Differences in Surface Expression of NspA among Neisseria meningitidis Group B Strains. Infect Immun 67 (11): 5664-75).

### Antigen presentation and vaccine formulation.

Earlier work has suggested that the form in which the antigens are presented is likely to be critical. The epitopes on membrane bound proteins are often dependent on maintenance of the correct tertiary structure and this in turn is frequently dependent on the hydrophobic membrane bound domains. It has been shown that the preparations of outer membrane proteins elicit immunity in humans only when presented in vesicle form (Zollinger WD, et al. 1979. complex of meningococcal group B polysaccharide and type 2 outer membrane protein immunogenic in man. J Clin Invest 63 (5): 836-48, Zollinger WD, et al. 1978. Safety and immunogenicity of a Neisseria meningitidis type 2 protein vaccine in animals and humans. J Infect Dis 137 (6): 728-39).

Single protein vaccines have been used in the field for decades and generally exhibit good stability. If presentation in the form of vesicles is required, to allow the antigens to remain membrane bound, stability and reproducibility may be difficult to guarantee. The immunogenicity and reactogenicity of outer membrane vesicles may vary with alterations in the amount of protein and LPS removed in the purification processes. A substantial body of experience in vesicle production has accrued in OMV vaccine manufacture, however, and the currently produced vaccines are subject to thorough quality control. Construction of entirely synthetic liposome vesicles may allow further optimization and standardization of such vaccines (Christodoulides M, et al. 1998. Immunization with recombinant class 1 outer-membrane protein from Neisseria meningitidis: influence of liposomes and adjuvants on antibody avidity, recognition of native protein and the induction of a bactericidal immune response against meningococci. Microbiology 144(Pt 11):3027-37). In other words, outer membrane proteins have been presented both, in vesicles and as pure expressed proteins, and the development of antibody responses has been modest. Main efforts so far have concentrated on intramuscular injection of meningococcal vaccine, leading to the production of systemic lgG. However, in meningococcal disease where invasion of the host is via the nasal epithelium, the production of secretory lgA may also be important.

### The N. meningitidis genome sequence

The genome sequences of MC58 (a serogroup B meningococcus) (Tettelin H, et al. 2000. complete Genome Sequence of Neisseria meningitidis Serogroup B Strain MC58. Science 287 (5459): 1809-15172) y and of Z2491 (a serogroup A strain) (Parkhill J, et al. 2000. complete DNA sequence of a serogroup A strain of Neisseria meningitidis Z2491. Nature 404 (6777):502-6173) were elucidated and published during 2000. The availability of the annotated gene sequences should have a dramatic influence on meningococcal vaccine research. While the MC58 genome sequencing was in progress, Pizza et al. began identifying the open reading frames that were predicted to encode either membrane bound, surface exposed or exported proteins. They identified 570 such ORFs, amplified them via the polymerase chain reaction and cloned them into *Escherichia coli* to allow expression of the encoded proteins as either His-tagged or glutathione S-transferase fusion proteins (Pizza M, et al. 2000. Identification of Vaccine Candidates Against Serogroup B Meningococcus by Whole-Genome Sequencing. Science 287 (5459): 1816-20). The 61% (350) of the selected ORFs were successfully expressed, those which failed to express were often those containing more than one hydrophobic trans-membrane domain (possibly excluding a number of outer membrane bound proteins). The recombinant proteins were purified and used to vaccinate mice. The immune sera were then assessed for surface binding to multiple meningococcal strains by enzyme linked immunosorbent (ELISA) assay and flow cytometry and for bactericidal activity against two strains using the serum bactericidal assay. Finally seven proteins were selected for further study on the basis of a positive response in all three assays. Trial vaccine formulations using a number of these proteins in combination with adjuvants have been shown to induce significant bactericidal tires against the homologous meningococcal strain (MC58) in mice, but none of the proteins induced SBA litres as high as an MC58 outer membrane vesicle vaccine (Giuliani MM, et al. 2000. Proceedings 12th IPNC. p. 22). On the other hand, there is some evidence that combinations of these proteins may exhibit higher immunogenicity in mice than single proteins (Santini L. et al. 2000. Proceedings 12th IPNC. p. 25). The numerous open reading frames which were excluded during this work, perhaps through failure of protein expression or modification of their immunological properties, may also have vaccine potential and require further investigation.

Vaccine components may be selected more effectively once an understanding of the contribution of individual antigens to the pathogenesis of *N. meningitidis* has been gained. The antigens themselves may make effective vaccine candidates or, alternatively, the attenuated mutants could be considered as vaccine constituents An important problem of meningococcal disease prevention and//or therapy is that no available vaccine to date confers a universal protection due to the heterogeneity of antigens used as vaccines so far.

### Description of the invention

This invention contributes to solve the problem mentioned before, by supplying pharmaceutical formulations containing a protein which sequence is highly conserved, even in different pathogenic bacterial genus. The technical objective that this invention pursues is the development of formulations with the ability to increase the systemic and mucosal host immune response against different new pathogens or a wider spectrum of existing ones. In the work object of the present invention it is reported, for the first time, the use of the NMB1796 protein as a component of a vaccine formulation with therapeutic or preventive character.

More specifically this invention is referred to pharmaceutical compositions, comprising this protein, aimed to prevent or treat any infection caused by a bacteria belonging to the Neisseria genus. In another preferred realization, these mentioned pharmaceutical compositions comprising previously said antigen, are useful for the prevention and treatment of diseases caused by *N. meningitidis* and *N. gonorrhoeae.* It is specifically, an object of this invetion, a pharmaceutical formulation where the NMB1796 protein is present in a range of 0.5-100 µg/dose, in an acceptable pharmaceutical formulation. Said formulation, comprises, optionally, a vaccine adjuvant capable to potentiate the immune response against the pharmaceutically active ingredient, the NMB1796 protein.

In another realization of this invention, pharmaceutical compositions might contain one or several antigens being of synthetic, recombinant or natural origin. In another realization of this invention, combined pharmaceutical compositions could contain polysaccharide antigens, including bacterial polysaccharides, and more specifically, *N. meningitidis* polysaccharides. Formulations of the present invention can contain conjugated protein-polysaccharides, being the polysaccharide of bacterial origin.

In one preferred realization of the present invention, pharmaceutical formulations containing NMB1796 also contain antigens of peptide nature, with the porpoise of expanding the protection spectrum induced by vaccines derived from said compositions.

This invention reveals pharmaceutical formulations, characterized by being a vaccine with the ability to elicit a protective immune response in the host organism against infections caused by bacteria of the Neisseria genus. More specifically, the pharmaceutical formulation of the present invention is a vaccine capable to elicit a protective response against inrfections caused by *Neisseria meningitidis* o *Neisseria gonorrhoeae.* Pharmaceutical formulations described herein are administered by parenteral or mucosal routes, including oral route.

In another preferred realization of the present invention, NMB1796 protein can be employed as adjuvant, or carrier of peptides, polysaccharides, or any other antigen with lesser immunogenicity, aiming to boost the immunogenicity of said elements. Example 11 shows that NMB1796 protein is capable to enhance the antibody levels against a viral-derived peptide when said peptide is conjugated to NMB1796. It is also comprised within the scope of the present invention to cover the use of protective determinants for a protein antigen given that they are inserted into the NMB1796 amino acid sequence, aiming to induce an enhanced immune response against such determinants, thus being part of new hybrid proteins present in a pharmaceutical composition.

In another preferred realization of the present invention, pharmaceutical compositions comprised in the present invention might contain NMB1796 protein fragments, capable to induce a protective response against the meningococcus or any other bacteria of the Neisseria genus. In a particular realization of the present invention, pharmaceutical compositions contain NMB1796 mimotopes or NMB1796 mimetic peptides generated by synthesis or recombinant DNA technology. The term "mimotope" describes herein any peptide being able to induce antibodies, and that they are combined with NMB1796 protein while being able to induce a protective immune response against Neisseria.

It is also a part of present invention the detection of meningococcal disease through the use of pharmaceutical components containing NMB1796, or the NMB1796 coding gene, identified as Seq. ID. No 3, in an independent way or among other components within a biological sample taken from an individual.

### Brief description of drawings

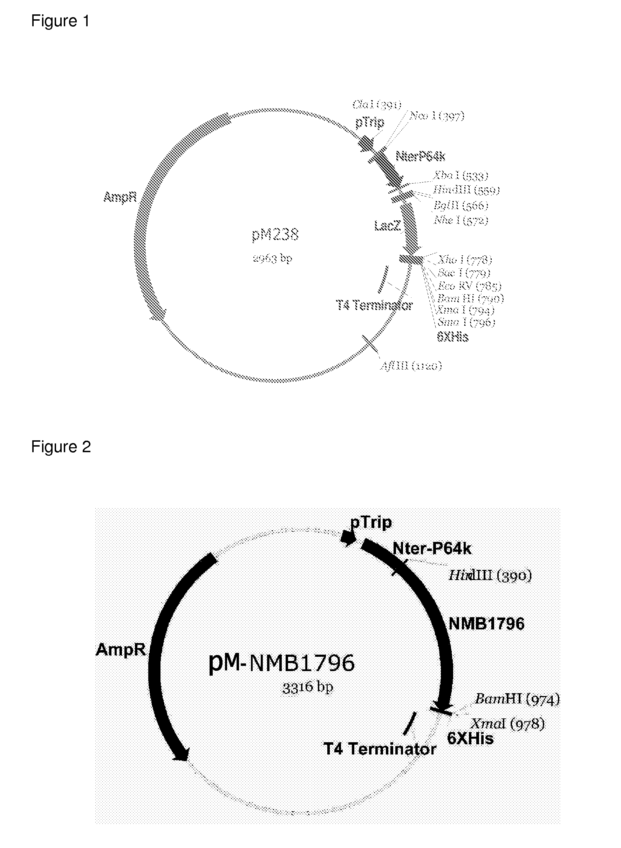

**Figure 1****.** Cloning vector pM238 employed in the cloning and expression of protein NMB1796.
**Figure 2****.** Final construction of nucleotide sequence of the gene *NMB1796* in pM238 vector.

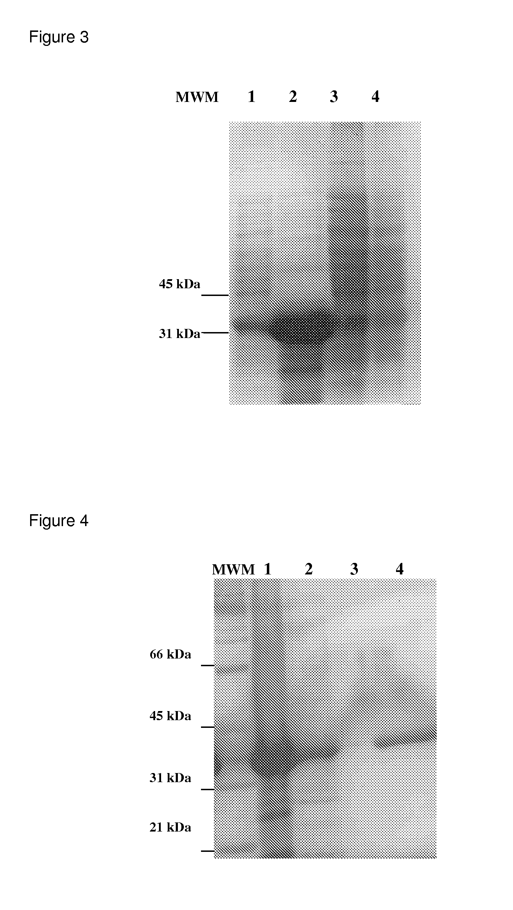

**Figure 3****.** SDS-PAGE analysis of fractions obtained from cellular disruption: WMW Molecular weight marker; lane 1, NMB1796 expression control; lane 2, pellet after rupture; line 3 supernatant after rupture; line 4 expression negative control.
**Figure 4****.** SDS-PAGE analysis of the different fractions of purification process of recombinant protein NMB1796: Lane 1, supernantant after 6M urea solubilization in adequate buffer; lane 2, unbound fraction (pass) to the matrix; lane 3, eluted fraction with 10 mM Imidazol; lane 4, eluted fraction with 100 mM Imidazol (purified protein). MWM: Molecular weight marker.

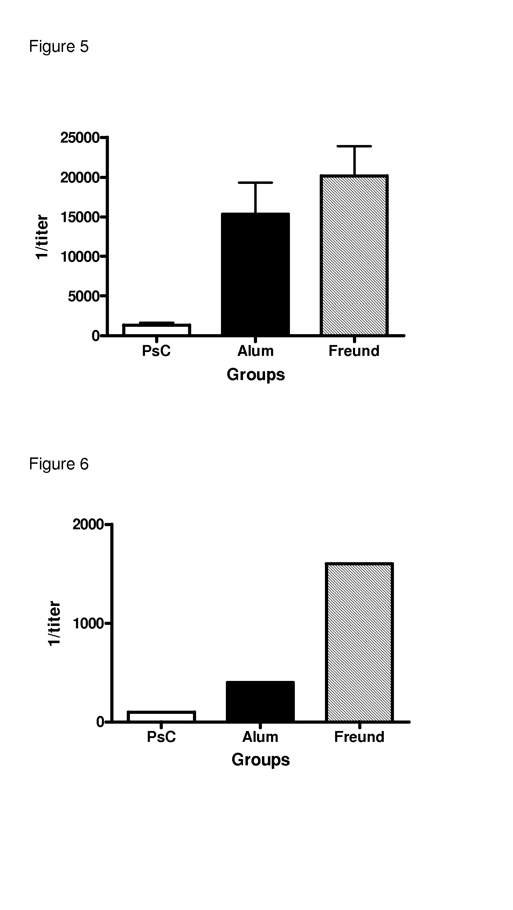

**Figure 5****.** Antibody levels (IgG) against recombinant protein NMB1796, obtained after mice immunization with the same antigen adjuvated with Freund's adjuvant (Freund), aluminum hydroxide (Alum) or *N. meningitidis* C polysaccharide by subcutaneous route. ELISA results are represented, as the inverse of the highest dilution that duplicates the value of pre-immune sera.
**Figure 6****.** Recognition of antigenic determinants present in *N. meningitidis,* strain CU385, OMVs using murine sera obtained after mice immunization with the same antigen adjuvated with Freund's adjuvant (Freund), aluminum hydroxide (Alum) or *N. meningitidis* C polysaccharide by subcutaneous route. Results are represented, as the inverse of the highest dilution that duplicates the value of pre-immune sera.

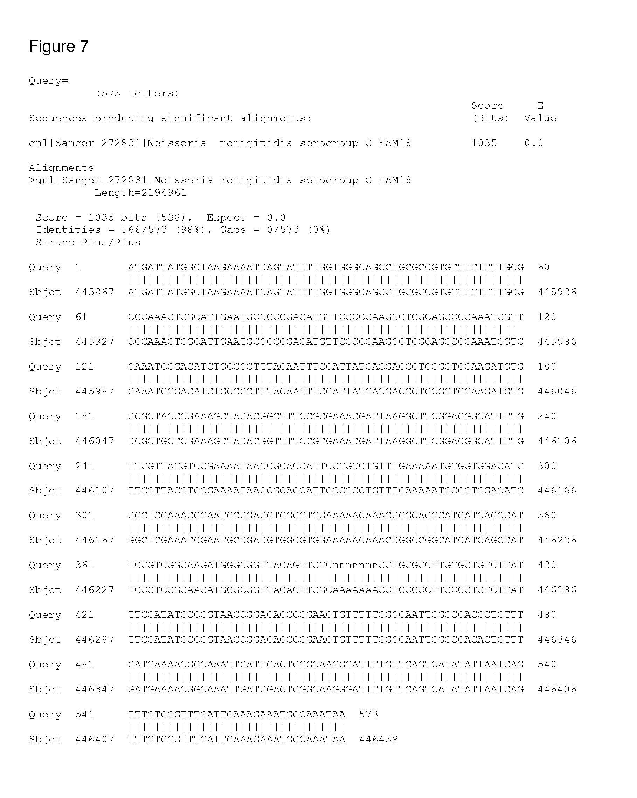

**Figure 7****.** Results of homology searches between NMB1796 protein ("query") and annotated sequences in genomes from different serogroups of *N. meningitidis* ("Sbjct") using the BLAST program.

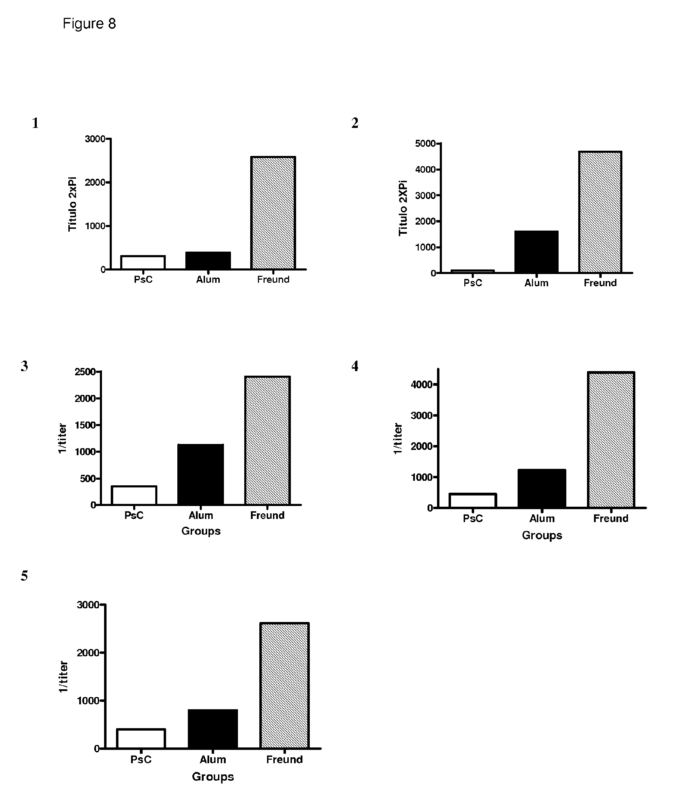

**Figure 8****.** Recognition of NMB1796 protein in five different strains of *N. meningitidis,* by sera elicited against the recombinant antigen adjuvated with Freund's adjuvant by subcutaneous route. Strains used were: **A,** Serogroup A; **C,** C:2a:P1.5; **B16B6,** strain B:2a:P1.2; **CU385,** strain B:4:P1.19,15; **NZ,** strain B4:P1.7b,4. In each strain, the membranes were incubated with **Poli** pools of polyclonal sera obtained by immunization with NMB1796; **Pi** pools of preimmnune sera of the same group. As negative control a polyclonal sera obtained against an heterologous antigen that contains the N-terminal fragment of P64k protein was used (**poli C-).** The arrow shows the immunoidentified protein NMB1796.

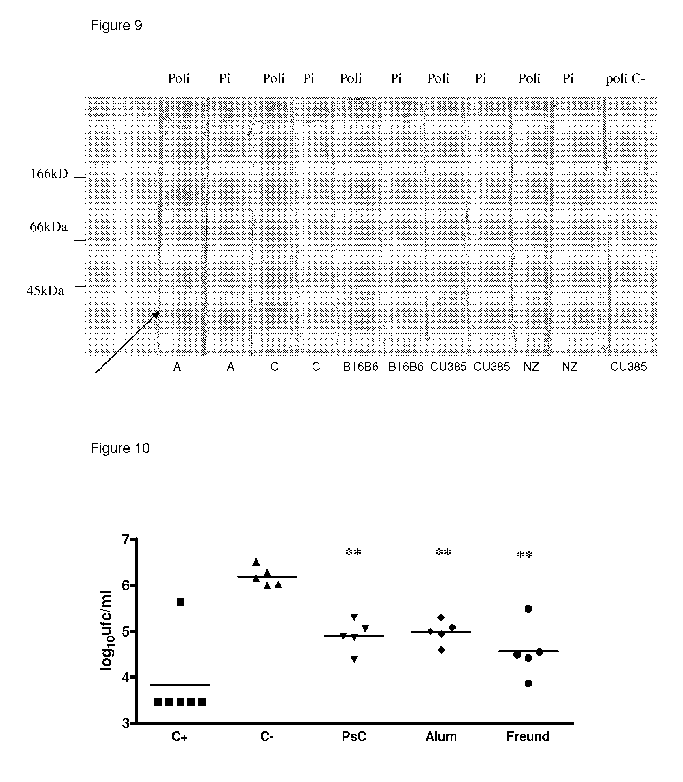

**Figure 9****.** Western Blotting recognition of NMB1796 protein in different strains of *N*. *meningitidis,* by sera elicited against the recombinant antigen adjuvated with Freund's adjuvant by subcutaneous route. Strains used were: **1,** Serogroup A; **2,** B:2a:P1.2; **3,** C:2a:P1.5; **4,** B:15:P1.7,16; **5,** B4:P1.7b,4. Sera elicited with other adjuvants had a similar profile. Results are expressed as the inverse of the highest dilution that duplicates the value of pre-immune sera.
**Figure 10****.** Meningococcal infection passive protection experiments in the infant rat model using sera elicited against the recombinant antigen adjuvated with *N. meningitidis* C polysaccharide (PsC), aluminum hydroxide (Alum), or Freund's adjuvant (Freund). Infection was done with strain Z4181. C-: pool of untreated animals, C+: hyper immune mice sera against outer memebrane vesicles of Z4181. The symbol ** represents a significant statistical difference in respect to the negative control (C-), Regarding to the levels of bacteraemia, these are expressed as colony forming units per mL (cfu/ml)

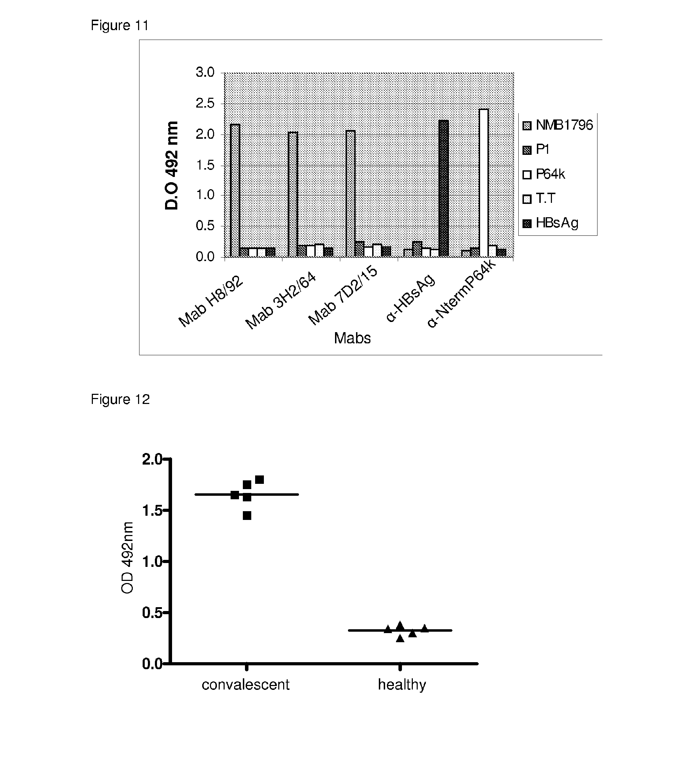

**Figure 11****.** Recognition of protein NMB1796 and a panel of un-related antigens by generated mAbs (mAbs H8/92, 3H2/64 y 7D2/15). P1, Class 1 protein *Neisseria meningitidis* strain B:4:P1.15; P64k, E3 subunit of pyruvate dehydrogenase from *Neisseria meningitidis;* T.T, tetanus toxoid; HBsAg, Hepatitis B surface Antigen. These results are shown as absorbance values (492 nm) in an ELISA-type assay.
**Figure 12****.** Recognition of NMB1796 protein by human convalescent sera from survivors of meningococcal disease. As negative control healthy donor sera were employed. Results are shown as the absorbance (492nm) in an ELISA type assay.

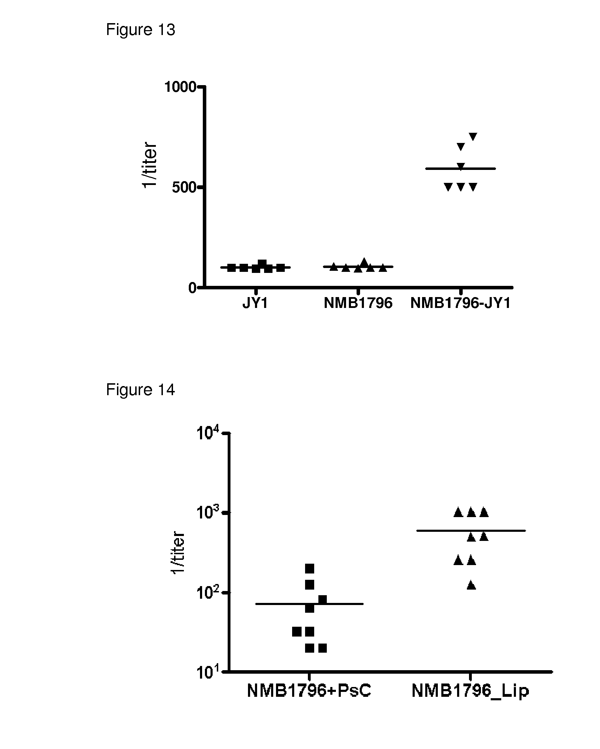

**Figure 13****.** JY1 anti-peptide titers from the sera of animals immunized with either free peptide (JY1), recombinant protein (NMB1796) or the conjugate JY1- NMB1796.
**Figure 14****.** Antibody Levels (lgA) against recombinant NMB1796 protein in pulmonary wash samples from intranasal immunized mice with a recombinant NMB1796 *N. meningitidis* C polysaccharide (NMB1796+PsC) or with the protein incorporated into liposomes (NMB1796_Lip).

### Detailed description of the invention/ Examples.

### Example 1. Detection of NMB1796 protein in serogroup B Neisseria meningitidis outer membrane vesicles preparations

With the aim of studying proteins that are present in serogroup B Neisseria meningitidis (strain B:4:P1.19,15) outer membrane vesicles, a bi-dimensional electrophoresis was carried out according to a method described elsewhere (Görg A, et al. 1985. Electrophoresis 6:599-604). Subsequently an enzymatic digestion was made upon the gel extracted proteins using trypsin (Promega, Madison, WI, U.S.). Peptides generated after digestion were extracted into solution by using micro columns (ZipTips, Millipore, MA, U.S.). For mass spectrometry analysis peptides were eluted from micro columns with acetonitrile 60%, formic acid 1% followed by an immediate application into nanotips (Protana, Denmark).

Measurements were carried out in a hybrid mass spectrometer with quadrupole and time of flight (QTof-2^{™}, Manchester, United Kingdom), fitted with an ionization source (nanoESI). Mass spectrometry data were acquired in a w/z range of 400-2000 in 0.98 seconds and using 0.02 seconds between scannings. Data acquisition and data processing were carried out using the MassLynx program (version 3.5, Micromass).

Protein identification based on mass spectrum data was carried out using the ProFound program (Zhang W and Chait BT. 2000. ProFound: an expert system for protein identification using mass spectrometric peptide mapping information. Anal Chem 72:2482-2489. http://prowl.rockefeller.edu/cgi-bin/ProFound). The search was subscribed to the genes and derived protein sequences contained in the SwissProt database (http://www.ebi.ac.uk/swissprot/) and NCBI (http://www.ncbi.nlm.nih.gov/), considering the oxidation of methionines, deamidation and carboxyamidomethylation of cysteines as possible modifications to be encountered.

Identification of proteins based on the mass spectra was carried out with the MASCOT program (Perkins DN, et al. 1999. Probability-based protein identification by searching sequence databases using mass spectrometry data. Electrophoresis 20:3551-3567. http://www.matrixscience.com/). Search parameters included cysteine modifications as well as oxidations and deamidations.

Starting from the analysis of results obtained from the identification of proteins present in preparations of outer membrane vesicles, the NMB1796 protein was selected to be evaluated as possible vaccine candidate, from which one peptide was identified by mass spectrometry.

### Example 2. Homology-based analysis of NMB1796 protein with gene products reported in databases.

For the identification of the NMB1796 protein, a sequence homology search was done in the NCBI data base employing the BLAST program (Altschul SF, et al. 1990. Basic local alignment search tool. J Mol Biol 215:403-410, http://www.ncbi.nlm.nih.gov/BLAST/). The results of this procedure indicated homology with, in addition to the corresponding protein in other serogroups of Neisseria, with the one in other microorganisms, including hypothetical proteins of Pseudomonas and Ralstonia genus.

The conservation of this protein among several microbial genes, has generated the denomination of an Orthologous protein group with a conserved domain in NCBI [gnl|CDD|26057 pfam03358, FMN_red, NADPH-dependent FMN reductase], presumable indicating a common phylogenetic origin from a common ancestor for all of them.

This is the first work where the value of this hypothetical conserved protein is pointed out as a valuable vaccine antigen, capable of inducing a protective humoral response after immunization with this protein in a vaccine formulation.

### Example 3. Cloning and expression of the nmb1796 gene, codifying for NMB1796 protein from N. meningitidis in Escherichia coli.

In order to clone and express the NMB1796 gene, the pM-238 cloning vector was employed. This vector allows the cloning to be carried out using different restriction enzymes and the generation of high expression levels of heterologous proteins in the form of inclusion bodies in *E. coli.*

The pM-238 vector (Figure 1) have the following elements: tryptophan promoter, gene segment codifying for the 47 amino acid stabilizing sequence from Nt-fragment of P64 kDa from *N. meningitidis* strain B:4:P1.19,15, sequence of bacteriophage T4 transcriptional terminator, and the sequence of the gene that confers resistance to Ampicillin as selection marker. This cloning vector also allows recombinant selection by the means of a blue/white color staining of transformed colonies, due to the presence of the beta-galatosidase lacZ alpha subunit.

From *nmb1796* coding gene nucleotide sequence (Example 1) a oligonucleotide primer pair (1796U and 1796L) was designed for amplification of said gene segment, avoiding the signal peptide coding region, from strain CU385 (B:4:P1.19,15) genomic DNA
*Xba*I
1796U: 5' **CCGGTCTAGATCTAGGAATGATTATGGC** '3
(No. Sequence identification: 1)
1796L: 5' **ATTCCCGGGATCCTTCAATCAAACC** 3'
*BamH*I
(No. Sequence ientification: 2)

For the prediction of signal peptide the SignalP World Wide Web server (http://www.cbs.dtu.dk/services/SignalP-2.0) was employed. After PCR amplification of the NMB1796-coding gene (Saiki R K, et al. (1988). S Primer-directed enzymatic amplification of DNA with a thermostable DNA polymerase. Science 239: 487-491) employing primers 1796U and 1796L, the PCR product was digested using Xba-I and Bam-HI restriction enzymes, and cloned into vector previously digested pM238 cloning vector. The final construction is showed in Figure 2, and the NMB1796 protein is expressed as a fusion protein to the Nt-segment of P64 kDa protein. Sequencing of the cloned gene *nmb1796* was carried out using ALFexpress II automatic sequencer (Termo Sequenase^{™} Cy^{™} 5 Dye Terminador Kit, Amersham Biosciences) and oligonucleotides 1573 (Seq. ID. No. 8) and 6795 (Seq. ID. No. 9) that bind the sequence of the P64 stabilizer and T4 transcriptional terminator, respectively. The plasmid generated herein was designated pM-NMB1796 for later use.

For the expression of the *nmb1796* gene the GC366 *E. coli* strain was transformed by the chemical method with the pM-NMB1796 plasmid (Figure 2). The expression experiment was carried out in minimal media (M9) (Miller JH. 1972. Experiments in Molecular Genetics, Cold Spring Harbor Laboratory Press, NEW York, USA) supplemented with glycerol 1%, triptone 1%, CaCl₂ 0.1 mM, MgSO₄ 1mM and ampicillin 50 µg/mL. Bacterial cultures were incubated 12 hours at 37 ºC and 250 rpm. Grown cultures were centrifuged and ultrasonic disruption of the cellular pellet was performed (IKA LABORTECHNIK). Fractions from pellet and supernatant were analyzed by SDS-PAGE (Laemmli UK. 1970. Cleavage of structural proteins during the assembly of the head of bacteriophage T4. Nature 277:680) plus stain with Coomassie Brilliant Blue R-250. The percent of expression was carried out by gel densitometry (LKB Bromma 2202 Ultrascan laser densitometer; Amersham Pharmacia Biotech, United Kingdom). The NMB1796 protein was obtained from the pellet fraction, being about 15% of total protein content of this fraction (Figure 3). Samples of cellular pellet were solubilized in Tris HCl buffer ( Tris HCl 20 mM pH=7.4) containing urea at different molar concentrations (2M, 4M, 6M and 8M). When the previous buffer with 6M was employed, the NMB1796 protein was solubilized. The supernatant, after solubilization, went through a metal affinity chromatography in order to achieve the purification of the protein of interest and a simple ws obtained where the molecule that migrates at the expected size is about 65% of total proteins. In Figure number 4 the electrophoretic pattern can be appreciated in some samples taken along the purification process. A last step of dialysis was performed before its evaluation in lab animals.

### Example 4. Evaluation of the immune response induced after immunization with NMB1796 protein by subcutaneous route.

To evaluate the immunogenicity of the protein NMB1796, an immunization experiment was designed and conducted in mice, where the NMB1796 protein was administered adjuvated with Aluminum Hydroxide, Freund's Adjuvant, or *N. meningitidis* C polysaccharide. With these preparations, female Balb/C mice (8-10 weeks-old) were immunized, once divided in 3 groups of 8 mice, each. Three immunizations were applied by subcutaneous route, with 7 days-interval in between. In Table 1 is described the composition of the groups:

**Table1: Balb/C mice groups employed in the immunization experiment**

| Group | Immunogen |
|---|---|
| 1 | 20µg NMB1796+ C polysaccharide |
| 2 | 20µg NMB1796 + Aluminum Hydroxide |
| 3 | 20µg NMB1796+ Freund's Adjuvant |

Antibody titers (IgG) against the recombinant protein and the homologous protein present in the bacterium were determined by an ELISA, in serum samples taken after the third dose. In Figure 5, the antibody titers against the recombinant protein of individual animals are shown. Specific antibody levels were detected right after the second inoculation (data not shown), being even higher after the last inoculation. Moreover, the immunoidentification by *Western blotting* was done, where the respective protein band was recognized (data not shown). The sera obtained after the immunization with the recombinant protein recognized the natural protein present in a preparation of outer membrane protein (OMP) of strain CU385. These results are represented in Figure 6.

### Example 5. Characterization of the sequence of the gene codifying for protein NMB1796 in different strains of N. meningitidis and Neisseria gonorrhoeae.

To analyze the conservation of the sequence of the gene codifying for the NMB1796 protein in the pathogenic species of the Neisseria genus a similarity search with the genomes of *Neisseria meningitidis* (serogroups A and B) and *Neisseria gonorrhoeae,* annotated in the NCBI data base, was done (NC 003116.1, NC 003112.1, NC 002946) employing the BLAST program (Altschul SF, et al. 1990. Basic local alignment search tool. J Mol Biol 215:403-410. http://www.ncbi.nlm.nih.gov/BLAST/). Figure 7 shows the results of the sequence comparison for those sequences that produce a significant aligment in each of the analyzed genomes. Those sequences have 100% identity in serogroup B, 99% identity in serogroup A and a 98% identity with *Neisseria gonorrhoeae,* with the sequence obtained for the gene that codifies for the NMB1796 protein (Seq. ID. No. 3). In addition, the sequence of the referred gene was determined for 3 Cuban isolates (Seq. ID. No. 5-7), which belong to serogroup B (B:4:P1.19,15) and a sequence alignment was done by using the ClustalX program (http://www.ebi.ac.uk/clustalw/). The results of the alignment show that there is a great conservation in the nucleotide sequence of the gene NMB1796 among the analyzed strains and in general in the *Neisseria* gender.

The use of the protein NMB1796 as a vaccine candidate, taking into account the high degree of similarity existing among the sequences previously mentioned, would allow the generation of an effective immune response, with a broad-spectrum protection (due to the cross reactivity) against the meningococcal disease.

### Example 6. Characterization of the immune response with broad-spectrum action induced by the immunization of Balb/C mice with the protein NMB1796.

To evaluate if the immunization with protein NMB1796 induced a response broadly cross-reactive with other strains of Neisseria, an ELISA was done. The polystyrene plates were coated with whole cells of 5 strains of Neisseria, which belong to different serotypes and serosubtypes. The plates were incubated with pooled sera obtained against the protein NMB1796, by two routes of immunization, as described in Example 4.

Figure 8 shows the recognition of antigens present in strains from serogroups A, B, and C from *N. meningitidis* by the sera elicited after the immunization with the recombinant NMB1796 protein adjuvated with Freund's Adjuvant. The rest of the sera had a comparable behavior in this assay.

Figure 9 shows the recognition by Western Blotting of a band that migrates at the expected size, by the sera elicited after the immunization with the recombinant NMB1796 protein adjuvated with Freund's Adjuvant. The same five strains of the previous assay were used.

### Example 7. Protection induced by the murine sera specific for protein NMB1796, against homologous and heterologous strains, in the infant rat model.

To determine the functional activity of the anti-sera obtained, a protection assay was conducted in the infant rat model for meningococcal infection. Twenty four rats (5-6 days old) were divided in groups of 6 rats each.

It was determined if the sera administered by intra-peritoneal route protected the rats from the infection caused by bacteria (strain Z4181), inoculated by the same route one hour later. The sera of each group were pooled and diluted 1/10 (in sterile PBS) before they were inoculated in infant rats. Four hours later, the animals were sampled and viable bacteria in their blood were counted.

To interpret the results, an Analysis of Variance (Anova) was done, followed by a Dunnet's Multiple Comparison Test, where the test groups were compared with the negative control. As can observed in Figure 10 the group receiving sera from mice immunized with NMB1796 adjuvated with the three adjuvants, did show statistically significant differences in comparison with negative control group, thus the antibody titers were protective in the infant rat model.

A similar assay was done infecting infant rats with strain CU385 (B:4:P1.19,15), and like in the previous experiment, antibodies from mice immunized with the protein mixed with three different adjuvant protected infant rat against meningococcal infection in the employed model (data not shown). A similar assay was done infecting infant rats with strains H44/48 and 120/90, isolated from Cuban patients, which serological classification is homologous to the strain B385. Moreover, challenge experiments were conducted with strain H44/76 (B:15:P1.7,16) from serogrop B. In all cases, the antisera containing antibodies elicited with the target NMB1796 protein was able to protect rats against meningococcal infection.

### Example 8: Immunogenicity of protein NMB1796 in an animal model of neonatal immunization.

With the aim to evaluate if the protein NMB1796 was able to induce an antibody response after vaccination in neonatal phase, an immunization experiment in neonatal OF1 mice. Immunization of 7 day old mice is considered a model that reproduces the characteristics of human humoral immune response during the neonatal phase for many antigens. Due to this fact, 3 groups of mice (6 mice/group), were immunized at days 7, 10 and 14 after birth. Mice form the first group received 10 µg of NMB1796 protein adjuvated with Alum (400 µg/dose). Second group mice received 10 µg of NMB1796 protein adjuvated with aluminum phosphate (400 µg/dose), while mice from the third group received 400 µg/dose of Aluminum hydroxide alone without antigen. At 21 days of age blood simples were taken and analyzed by ELISA, in order to measure the antibody levels against NMB1796. The anti-NMB1796 titers detected in all three groups of mice is shown below.

**Table 2. Anti-NMB1796 antibody titers detected in three groups of OF1 mice immunized in neonatal phase**

| Group | Administered Formulation | MGT* |
|---|---|---|
| 1 | NMB1796+ Al(OH)₃ | 4876 |
| 2 | NMB1796+ AlPO₄ | 5699 |
| 3 | Al(OH)₃ | 50 |

| | | |
|---|---|---|
| *: MGT, medium geometric titers of IgG anti-NMB1796 antibody titers detected in mice of 21 days of birth. | | |

As it can be appreciated in Table 2 both formulations containing NMB1796 protein and human-use-accepted adjuvants elicited significant antibody levels for an animal model with a remarkable immaturity on its immune system, thus suggesting the fact. That this vaccine antigen can be immunogenic alter its administration into human neonates, and in turn being able to confer immunity against the Meningococcus at this early moment of life.

### Example 9

### Generation of monoclonal antibodies against protein NMB1796 able of mediating the bactericidal activity against Neisseria meningitidis

To generate monoclonal antibodies (mAbs) specific against protein NMB1796, and study the functional ability of mediating bactericidal activity against homologous and heterologous strain of *N. meningitidis,* an immunization schedule was conducted with a preparation of protein NMB1796 with 85% purity (Example 3). The immunization was done in Balb/C (H-2^{d}, female, 5-6 weeks old) and 4 doses were applied as follows: On days 0, 15 and 30 of the immunization routine, 10 µg of antigen NMB1796 per mouse (total volume 100 µl), were administered by subcutaneous route, emulsified with Freund's Adjuvant; on day 50, 10 µg of antigen per mouse in Phosphate Buffered Saline (140 mM NaCl, 270 mM KCI, 1.5 mM KH₂PO₄, 6.5 mM Na₂HPO₄ x 2H₂O, pH 7.2) were administered by intra-peritoneal route. Blood extractions were done on days 0 and 45.

Splecnocytes from the animal with the highest titer, measured by an indirect ELISA using protein NMB1796 as the coating antigen (Example 3), were fused with X63 Ag8 653 mouse myeloma cells. The resulting hybridomas were isolated and screened according to standard procedures (Gavilondo JV. 1995. Anticuerpos Monoclonales: Teoría y Práctica, Elfos Scientiae, La Habana, Cuba).

The reactivity of the antibodies secreted by the hybridomas directed to protein NMB1796, as well as their cross-reactivity non-related antigens, was tested by an indirect ELISA employing 5 µg/ml of each antigen, and the same concentration of each mAbs to be assayed. Figure 11 shows the results obtained in this experiment, all together 2 positive clones were obtained (mAbs H8/92, 3H2/64 y 7D2/15) which specifically recognized protein NMB1796, and do not react neither with the amino acid sequence corresponding to the N-terminal of P64k, nor with the rest of the non-related antigens assayed.

To determine the ability of the mAbs generated against protein NMB1796 to mediate a bactericidal response against homologous and heterologous strains of *Neisseria meningitidis* a bactericidal test was performed. The bactericidal antibody titer was expressed as the reciprocal of the highest dilution of the antibodies tested that was able of killing 50% or more bacteria; two of the mAbs (3H2/64 y 7D2/15) achieved bactericidal titers higher than 1:128 against the homologous strain B:4:P1.19,15 and one (H8/92) a titer higher than 1:80. They also showed titers higher than 1:64 against the heterologous strains B:15:P1.7,16 and C:2a:P1.5 respectively.

### Example 10

### Characterization of the target regions of the murine immune response against protein NMB1796

In order to identify the regions in the protein, which are more frequently recognized by the murine anti-sera generated against the recombinant antigen a SPOTScan assay was done. A set of overlapping peptides that span the sequence of the protein was synthesized on a cellulose membrane, which was incubated with pooled sera diluted 1:100. The antigen-antibody reaction was detected by the incubation with a conjugate anti-murine immunoglobulin G- alkaline phosphatase, followed by the addition of a solution that contained the substrate Bromo-chloro-indolyl-phosphate. Several antigenic regions common within the protein were observed, no matter the preparation that was employed for the immunization (data not shown).

### Example 11. Recognition of the NMB1796 protein by human sera.

A collection of human sera, coming from convalescent individuals was employed in this study, which was performed by ELISA. The plates were coated with protein NMB1796, obtained by preparative electrophoresis (5 µg/ml). Alter blocking the plates with 3% skim milk powder in PBS containing Tween-20, the sera were diluted (1:50) in the same solution and were incubated in the plates. The immunoassay continued as it has been widely reported. Healthy donor sera were employed as negative controls. In addition, pooled sera from individuals vaccinated with a recombinant vaccine against Hepatitis B was used a non-related control (data not shown).

Figure 12 shows the results obtained with 5 convalescent's sera in this assay. It can be seen that the human sera recognized the protein, which indicates that it is expressed during the meningococcal infection and it is immunogenic.

### Example 12. Protein NMB1796 as a carrier for a peptide.

To demonstrate the carrier capacity of the recombinant protein NMB1796, it was conjugated to a 15 mer synthetic peptide, derived from the V3 region of protein gp120 from HIV-1, isolate JY1. The conjugation was done by the glutaraldehyde method. Free JY1 peptide, the recombinant protein NMB1796 and the conjugate JY1- NMB1796, were administered to adult mice in a 3-dose schedule, where the immunogens were emulsified with Freund's Adjuvant. Two weeks after the third dose, serum samples were obtained from the immunized animals, and the samples were analyzed by ELISA to determine the anti-peptide antibody titers. To do that, the plates were coated with free peptide (20µg/ml) and the immunoassay continued as it has been previously described. The results of the experiment (Figure 13) show the carrier capacity of protein NMB1796, able of significantly potentiate the antibody response against peptide JY1, after their conjugation.

### Example 13. Evaluation of the immune response induced after immunization with NMB1796 protein by mucosal route.

To evaluate the immunogenicity of the protein NMB1796, an immunization experiment was designed and conducted in mice, where the NMB1796 protein was administered encapsulated into liposomes or adjuvated with *N. meningitidis* C polysaccharide. Liposomes were obtained by dehydration-rehydration as previously described (Carmenate T, et al. (2001). Recombinant Opc protein from Neisseria meningitidis reconstituted into liposomes elicits opsonic antibodies following immunization. Biotechnol. Appl. Biochem. 34: 63-69). With these two preparations, female Balb/C mice (8-10 weeks-old) were immunized. Three doses of 50 µg via intranasal route were applied, with 15 days-interval in between. In order to analyze the immune response at mucosal level, IgA antibody levels were measured in pulmonary washes of immunized animals. In Figure 13 the detected IgA antibody levels are shown for the two groups evaluated.

## Claims

1. Pharmaceutical composition **characterized by** containing the protein NMB1796 identified as Seq. ID. No 4.

2. Pharmaceutical composition according to claim 1 for the prevention or treatment of infections caused by bacteria from the *Neisseria* genus.

3. Pharmaceutical composition according to claim 1 for the prevention or treatment of infections caused by *Neisseria meningitidis or Neisseria gonorrhoeae.*

4. Pharmaceutical composition according to claim 1, where protein NMB1796 is present in the 0.5-100 µg/dose range, in a pharmaceutically accepted formulation.

5. Pharmaceutical composition according to claim 4, **characterized by** containing an optional vaccine adjuvant.

6. Pharmaceutical composition according to claim 1, **characterized by** containing in addition one or several antigens of different nature obtained by recombinant, synthetic, or natural means.

7. Pharmaceutical composition according to claim 6, **characterized by** containing polysaccharide antigens, including bacterial polysaccharides.

8. Pharmaceutical composition according to claim 7, **characterized by** containing *Neisseria meningitidis* capsular polysaccharides.

9. Pharmaceutical composition according to claim 6, **characterized by** containing protein-polysaccharide conjugates in which the polysaccharide component is a bacterial polysaccharide.

10. Pharmaceutical composition according to claim 6, **characterized by** containing peptide antigens.

11. Pharmaceutical composition according to claim 1 **characterized by** being a vaccine able to generate in the recipient organism a protective response against infections caused by bacteria from the *Neisseria* genus.

12. Pharmaceutical composition according to claim 11, **characterized by** being a vaccine able to generate in the recipient organism a protective response against infections caused by *Neisseria meningitidis or Neisseria gonorrhoeae.*

13. Pharmaceutical composition according to claim 1, to administered by parenteral or mucosal routes.

14. Pharmaceutical composition according to claim 1, **characterized by** the use of NMB1796 protein alone, combined or fused, as an adjuvant or carrier for antigens of different nature.

15. Pharmaceutical composition **characterized by** containing peptide fragments, or mimetic peptides of NMB1796 protein antigen, in pharmaceutically accepted formulations.

16. A method for diagnosing an infection caused by bacteria from Neisseria genus, and comprising the detection of NMB1796 protein or their fragments, either in an independent way or in combination with other components, in a biological sample from an individual.

17. A method for diagnosing an infection caused by bacteria from Neisseria genus, and comprising the detection of the NMB1796 coding gene, identified as Seq. ID. No 3, either in an independent way or in combination with other components, in a biological sample from an individual.
